# EUROPEAN PATENT SPECIFICATION

(11) **EP 1 841 814 B1**
(45) Date of publication and mention of the grant of the patent: **18.11.2009**
(21) Application number: 06701563.6
(22) Date of filing: 17.01.2006
(51) Int. Cl.: C08J 7/04, A61L 29/08, A61F 2/02, G02C 7/04

(54) **A METHOD OF COATING A POLYMER SURFACE WITH A POLYMER CONTAINING COATING AND AN ITEM COMPRISING A POLYMER COATED POLYMER**
VERFAHREN ZUM BESCHICHTEN EINER POLYMEROBERFLÄCHE MIT EINER POLYMERHALTIGEN BESCHICHTUNG UND GEGENSTAND, ENTHALTEND EIN POLYMERBESCHICHTETES POLYMER
PROCEDE PERMETTANT D'ENROBER UNE SURFACE POLYMERE AVEC UN REVETEMENT CONTENANT UN POLYMERE ET UN ELEMENT COMPRENANT UN POLYMERE ENROBE D'UN POLYMERE

(30) Priority: 17.01.2005 DK 200500085
(43) Date of publication of application: 10.10.2007
(73) Proprietor: Biomodics, 2800 Lyngby (DK)
(72) Inventor: BENTER, Maike, DK-4000 Roskilde (DK); ALM, Martin, DK-2670 Greve (DK)
(74) Representative: Hegner, Anette
(86) International application number: PCT/DK2006/050003
(87) International publication number: WO 2006/074666

(56) References cited:
- EP-A- 0 706 821
- WO-A-01/83873
- WO-A-98/36784
- WO-A2-2004/091571
- DE-A1- 10 238 559
- US-A- 4 291 133
- US-A1- 2002 155 241
- US-A1- 2003 215 572
- US-B1- 6 506 437

## Description

### TECHNICAL FIELD

The invention relates to a method of coating a surface of a polymer with a polymer containing coating and an item comprising a coated polymer. The method of the invention may e.g. be useful in production of items with a biocompatible surface, such as a medical device e.g. for use in contact with the human body and laboratory utensils for use in biological tests.

### BACKGROUND ART

A large number of prior art publications disclose various methods of applying a polymer coating onto another polymer. One prior art method Includes a simple mechanical application, including dissolving the coating polymer in a solvent, in general an organic solvent and applying the solution onto the substrate and allowing the solvent to evaporate.

Another method includes melting the coating polymer and applying it onto the substrate using an extrusion process.

Yet another method comprises the step of applying the polymer coating in a plasma process, including the steps of placing the substrate in a plasma chamber e.g. a plasma chamber as disclosed in WO 0044207 or those utilizing the electrode system described in EP 741404, introducing monomer for the polymer coating into the plasma chamber and generating a plasma, whereby the monomers will be deposited and polymerized to form the polymer coating. Known plasma generating methods include the methods as described in EP 1286382, WO 02094906, WO 0235895 and US 5935455.

WO 01/83873 describes a method for coating or impregnating a substrate such as a textile fabric with a treatment material such as a water or stain resistant coating comprises the steps of (a) combining a substrate and an inert transfer vehicle (e.g., a sheet material) in a pressure vessel, the inert transfer vehicle carrying the treatment material; (b) pressurizing the vessel with a carbon dioxide medium; and then (c) agitating the substrate and the inert transfer vehicle together in the pressure vessel with the substrate and the inert transfer vehicle at least periodically immersed in the carbon dioxide medium so that the treatment material is transferred from the inert transfer vehicle to the substrate. The treatment material may comprise polymer segments which are dissolvable in supercritical carbon dioxide.

### DISCLOSURE OF INVENTION

Although a number of techniques for coating a polymer with another polymer are known, there is still a need for alternative methods, and in particular a simple method which can be used for applying a thin polymer coating onto another polymer.

The objective of the present invention is therefore to provide a novel method of coating a surface of a solid polymer with a coating comprising a polymer, which method is simple, reproducible and wherein the polymer coating can be relatively thin and preferably homogeneous.

Another objective is to provide a method of coating a surface of a solid polymer with a coating comprising a biocompatible polymer, preferably the method includes applying a thin coating of a biocompatible polymer.

Yet a further objective is to provide an economical method of providing a polymer surface with bio-repelling properties (low adherence to biocomponents).

The objects of the invention are achieved by the invention described in the accompanying claims and as described in the following.

The method of the present invention of coating a surface of a solid polymer substrate with a polymer containing coating is defined in claim 1 and comprises the steps of
a) providing a coat polymer for the coating, and dissolving said coat polymer in a first solvent comprising at least 50% by weight of an organic solvent
b) placing said solid polymer substrate in a reaction chamber
c) introducing said polymer coat solution into the reaction chamber,
d) introducing carbon dioxide into the reaction chamber, and
e) depositing the coating onto the solid polymer substrate surface,
wherein the carbon dioxide during the deposition step is in its liquid state at a pressure up to 7.4 MPa.

The term "coat polymer" designates the polymer to be coated onto the solid polymer substrate.

The steps a)-d) of the method of the invention may be performed in any order. In one embodiment the coat polymer is fully dissolved prior to being introduced e.g. by injection into the reaction chamber. Thereby a complete dissolution can be secured and thus an optimal use of the coat polymer. As the coat polymer may be a relatively expensive polymer, it may be economically beneficial to secure that the complete amount of coat polymer is available for the coating process.

In one embodiment the coat polymer is fully or at least partly dissolved prior to being introduced e.g. by injection into the reaction chamber. This method may be beneficial if the coat polymer is not too expensive, and/or if the first solvent is expensive and/or difficult to recover for reuse. Depending on the coat polymer and the first solvent it may not be necessary to fully dissolve the coat polymer prior to introduction into the reaction chamber, because the coat polymer may be further dissolved within the reaction chamber. For the specific combination of the solution and coat polymer the skilled person will be able to optimize the dissolution step.

In one embodiment it is preferred that the step of dissolving the coat polymer in a first solvent is performed prior to bringing it into contact with the solid polymer substrate. Thereby the risk of undesired dissolution is reduced.

It has thus surprisingly been observed that the risk of dissolving the solid polymer substrate, even in situations where the first solvent is also a solvent for said solid polymer substrate, is extremely low, provided that the first solvent is not added directly onto the solid polymer substrate prior to the introduction of the carbon dioxide. If carbon dioxide in its liquid and/or gaseous state is added to the reaction chamber prior to the introduction of the first solvent or the polymer coat solution, the risk of undesired dissolution of the solid polymer substrate has found to be negligible.

Thus, in one embodiment the first solvent and the coat polymer are introduced separately into the reaction chamber and are first brought into contact and dissolved within the reaction chamber.

The solid polymer substrate may in principle be placed within the reaction chamber at any time prior to the deposition step. For practical reasons, however, the solid polymer substrate will almost always be introduced into the reaction chamber prior to introduction of carbon dioxide.

In one embodiment of the method of the invention, the polymer coat solution is injected into the reaction chamber simultaneously with introduction of carbon dioxide into the chamber. The polymer coat solution and the carbon dioxide may e.g. be injected via separate injection channel or they may be injected via the same injection channel. In one embodiment the polymer coat solution is injected using an injection needle, and the carbon dioxide is injected via a pressure pipe connected to a carbon dioxide pressure tank.

In one embodiment the carbon dioxide is mixed with the polymer coat solution prior to injection into the reaction tank.

In one embodiment of the method of the invention, the method comprises the steps of placing the solid polymer substrate in the reaction chamber, the carbon dioxide is introduced into the reaction chamber to raise the pressure to at least 0.2 MPa, where after the polymer coat solution is injected into the reaction chamber. In this method the pressure within the reaction chamber may e.g. be raised to at least 0.5 MPa, such as to at least 1.0 MPa, such as at least 1.5prior to injecting the polymer coat solution into the reaction chamber. It is preferred that the pressure is kept below 7.4 MPa, such as below 7.0 MPa, such as between 5 and 5.5 MPa, because the higher the pressure is within the reaction chamber, the higher strength is needed of the reactor chamber, and thus the more expensive the reactor will be. It is thus beneficial for the method of the invention that it can be carried out under such relative low pressure comparing to other prior art methods. By changing the pressure in the reactor the density of the carbon dioxide will be changed as well, and by this change of density the dissolving power of the carbon dioxide will also change. For a specific coat polymer the skilled person will be able to find an optimal pressure based on the teaching given herein.

By introducing the polymer coat solution after at least some of the carbon dioxide has been introduced into the reaction chamber, preferably so that the pressure is at least higher than atmospheric pressure, it appears that the utilization of the coat polymer is very high.

In one embodiment of the method of the invention, it is desired that the reaction chamber at the time of introducing the polymer coat solution into the reaction chamber, comprises carbon dioxide in its liquid state. Thereby the deposition will start immediately after introduction of the polymer coat solution.

In a preferred embodiment the method of the invention comprises the step of dissolving the coat polymer in a first solvent, followed by mixing said polymer coat solution with carbon dioxide in gas form where after the polymer coat solution carbon dioxide mixture is injected into the reaction chamber.

In another preferred embodiment the method of the invention comprises the step of dissolving the coat polymer in a first solvent, followed by mixing said polymer coat solution with carbon dioxide in liquid form where after the polymer coat solution carbon dioxide mixture is injected into the reaction chamber.

The solid polymer substrate may in principle be any type of polymer substrate. In general it is preferred that the solid polymer substrate is of a polymer material which has a simple shape, e.g. produced by injection molding. The solid polymer substrate is a polymer composition selected from the group consisting of silicone polymers, thermoplastic elastomers, rubbers, polyolefins, polyesters, polystyrene, polyacrylates, polyethers, polyurethane, polycarbonate, thermoplastic vulcanisates, polyurethane, epoxy polymers, thermoset polyimide and mixtures thereof.

The weight % refers to the dry weight (drying at 50 °C until constant weight) of the polymer.

In a preferred embodiment the solid substrate is of a polymer composition comprising at least 10 %, such as at least 20%, such as at 40%, such as at least 60 %, such as at least 80% by weight of a thermoplastic elastomer selected from the group consisting of a random copolymer, a block copolymer, more preferably TPE, even more preferably selected from the group consisting of SEBS, SBS, SIS, TPE-polyether-amide, TPE-polyetherester, TPE-urethanes, TPE PP/NBR, TPE-PP/EPDM, TPE-vulcanisates and TPE-PP/IIR.

In one embodiment of the method of the invention the solid polymer substrate is of a polymer composition comprising at least 10 %, such as at least 20 %, such as at least 40 %, such as at least 60 %, such as at least 80 % by weight of a rubber selected from the group consisting of butadiene rubber, isoprene rubber, nitril rubber, styrene-butadiene rubber, latex and urethane rubber.

In one embodiment of the method of the invention the solid polymer substrate is of a polymer composition comprising at least 10 %, such as at least 20 %, such as at least 40 %, such as at least 60 %, such as at least 80 % by weight of an polyolefin selected from the group consisting of polyvinyls such as polyvinylpyrrolidone, polyethylene, polypropylene, polybutylene including its isomers.

In one embodiment of the method of the invention the solid polymer substrate is of a polymer composition comprising at least 10 %, such as at least 20 %, such as at least 40%, such as at least 60 %, such as at least 80 % by weight of silicone polymers selected from the group consisting of dimethyl polysiloxane, methylphenyl polysiloxane, fluorosilicone rubber, silicone esters, polysiloxanes, polysilanes, chlorosilanes, alkoxysilanes, aminosilanes, polysilanes polydialkylsiloxanes, polysiloxanes containing phenyl substituents, said polymers of the silicone polymer composition optionally being vinyl-functionalized and/or optionally being partially or fully fluorinated.

In one preferred embodiment the solid polymer substrate is of a silicone polymer. This embodiment is particularly preferred for the production of items which are to be used in contact with the human or animal body, such as a catheter, an implant and a contact lens.

Its has thus been observed that the treatment in the reaction chamber using liquid carbon dioxide has a sterilizing effect on the substrate, which thus makes it even more beneficial for items which are to be used in contact with the human or animal body.

In one preferred embodiment the solid polymer substrate is of polycarbonate and/or polyethylene and/or polypropylene and/or polystyrene. This embodiment is particularly preferred for the production of a laboratory utensil, such as test tube/plate e.g. an Elisa plate, a flow cell, a slide, and a stirrer. This embodiment may also be desired in the productions of other items, such as a contact lens, a synthetic blood vein, a catheter, a hip implant

The solid polymer substrate should preferably not contain flow stress from the fabrication/manufacturing process, such as injection molding, since such stress may give rise to crack formation when the solid polymer substrate is placed in carbon dioxide. A preferred solid polymer substrate is a polystyrene substrate, but often polystyrene comprises flow stress symptoms e.g. if the solid polymer substrate is made by injection molding. The flow stresses may therefore in one embodiment be minimized or even eliminated by annealing (pre-heat treatment as disclosed below).

The solid polymer substrate may comprise fillers and additives. In one embodiment the solid polymer substrate comprises up to 40 % by weight, preferably up to 30 % by weight, preferably up to 20 % by weight, preferably between 2 and 10 % by weight of fillers and/or additives. The fillers may e.g. be particles or fibres, such as in the form of minerals or organic fillers. Preferred fillers include fillers selected from the group consisting of carbon black, carbon fibers, granulated rubber tires, silica, metals, metal oxides, mixed metal oxides, glass beads or glass fibers. Preferred additives include additives selected from the group consisting of adhesion promoters for 2K-constructions, process and plasticizing oils, antioxidants and pigments.

The coat polymer has another composition than the solid polymer substrate. In one embodiment the coat polymer has other properties relating to its hydrophilic and/or oleophilic character.

In one embodiment the coat polymer comprises a polymer which is more hydrophilic than the solid polymer substrate. This embodiment is particularly useful in the production of items for use in contact with the human or animal body.

In one embodiment the coat polymer comprises a polymer which is more hydrophobic or more oleophobic than the solid polymer substrate. This embodiment is particularly preferred for the production of a laboratory utensil.

Most preferably the coat polymer comprises a polymer which is both more oleophobic and or more hydrophilic than the solid polymer substrate, thereby the coating will provide the solid polymer substrate with improved properties both with respect to wettability, which is important in some applications e.g. such as in the production of catheters and contact lens, and lipid repelling properties which result in non-sticking of biocomponents (also called bio-repelling properties), such as cells, proteins, enzymes and similar.

The coat polymer may in one embodiment comprise one or more polymers with anti oxidizing properties. Thereby the solid polymer substrate may be provided with a surface which is very useful in contact with the human and/or animal body. This anti oxidizing surface may also be useful in other items such a laboratory utensils.

The coat polymer may in one embodiment comprise one or more polymers with electrically conductive properties. These properties may e.g. be used in laboratory utensils.

The method of the invention provides the possibility of applying a coating with a relatively high molecular weight. Thus, in one embodiment the coat polymer has an average number molecular weight of at least 5,000, preferably at least 10,000, such as between 20,000 and 500,000, preferably less than 300,000, such as less than 100,000.

According to the method of the invention it was found that the coat polymer, even with this relatively high molecular weight may be applied onto the surface without any substantial change of its chemical structure. The coating is thus not bonded covalently to the solid polymer.

It is believed that the strength of the bonding between the solid polymer substrate and the coating is due to a combination of physical bonding and mechanical bonding (interlocking obtained by partial interdiffusion), i.e. the coating is at least partly penetrating into the surface of the solid polymer substrate and thereby anchored in the solid polymer substrate.

The coat polymer comprises one or more of the polymers selected from the group consisting of PTFE, phospholipids containing polymers, such as 2-methacryloyloxyethylphoiphorylcholine (MPC) containing polymers; butyl metacrylate containing polymers (PBMA); poly(2-hydroxyethyl metacrylate) (PHEMA) containing polymers; polydimethylsiloxane (PDMS); poly(ethylene glycol) (PEG); vinylpyrrolidone containing polymer, such as poly(vinylpyrrolidone) (PVP) and copolymers thereof.

Most preferred for obtaining bio-repelling properties the coat polymer comprises one or more polymer having metacrylate backbone, such as butyl metacrylate containing polymers (PBMA) and poly(2-hydroxyethyl metacrylate) (PHEMA) containing polymers. Some of these bio-repelling polymers are relatively expensive, e.g. MPC, and the method of the invention is therefore highly beneficial when applying a coat polymer comprising one or more of these polymers, because the necessary amount of polymer for the coating is highly reduced compared with prior art methods.

In one embodiment non-used coat polymer is purified and reused.

The first solvent may in principle be any type of solvent comprising at least 50% by weight of organic solvent. As the first solvent will be driven out of the applied coating using the carbon dioxide, there will be no residue of the solvent when the application of the coating is terminated. Therefore also organic solvent may be used, without leaving residue in the final product. The first solvent may be recovered and reused or it may be burned off.

Suitable first solvents include solvents selected from the group consisting of cyclohexanones; alcohols, such as methanol, ethanol butanol, isopropanol and propylene glycol; acids of alcohols, esters such as ethyl acetate, xylene, toluene and mixtures thereof optionally including water.

The first solvent comprises at least 50 by weight of an organic solvent, such as at least 60% by weight, such as at least 75 % by weight, such as at least 90 % by weight, such as essentially all of the solvent being an organic solvent.

In one embodiment the first solvent comprises a mixture of water and one or more organic solvents.

In one embodiment the first solvent comprises a mixture of carbon dioxide and one or more organic solvents.

The use of carbon dioxide in combination with water and/or one or more organic solvents as the first solvent may preferably be desired when the coat polymer is of a polar nature. The solubility properties of carbon dioxide can thus be significantly modified by adding even small amounts of another solvent.

For the specific coat polymer the skilled person may find a useful first solvent e.g. by using the solubility theory provided by Charles Medom Hansen "A users guide to Hansen's solubility parameters" (2000). In one embodiment the first solvent is selected according to Hansens solubility parameters (HSP) to have an HSP which is less than the radius of the coat polymers.

In one embodiment the method further includes dissolving or dispersing at least one additional component in the first solvent, the additional component preferably being selected from the group consisting of pigments; anti-thrombotic agents, such as proteins and peptides; anti-microbial agents, such as silver salts; antioxidants, such as Octadecyl 3,5-di-t-butyl-4-hydroxyhydrocinnamate. Thereby additional properties may be provided. The additional agents dissolved in the first solvent may penetrate deeper into the solid polymer substrate than the coat polymer due to their smaller sizes. It may thus be possible to apply a coating of a coat polymer and simultaneously partly or totally impregnate the solid polymer substrate with one or more additional components.

In one embodiment the method of the invention includes dissolving a coat polymer and a cross linking agent for said polymer in the first solvent. The method additionally includes a step of cross linking the coat polymer after it has been applied to the solid substrate. In this embodiment the coat polymer may preferably be selected from the group of HEMA, NVP, vinylacetate and acrylates. The cross linking agent may preferably be activatable using heat or irradiation (e.g. IR or UV irradiation). The cross linking step may thus preferably include subjecting the coated solid substrate for an activating step preferably selected form the group of activation by heat or irradiation or both.

In one embodiment the solid polymer substrate is subjected to a pretreatment prior to the step of applying the coating. The pretreatment may comprise an extraction step including extraction of residual monomers, additives, oils and water from the solid polymer substrate. The extraction step may e.g. be performed using an extraction process as described in WO 03068846.

The extraction step may in one embodiment be performed by subjecting the solid polymer substrate to one or more of the treatments, a heat treatment, a vacuum treatment, a treatment with a supercritical solvent and at treatment with liquid carbon dioxide.

In one embodiment at least 0.05% by weight, such as at least 0.1 % by weight, such as at least 0.5 % by weight, such as at least 1 % by weight, such as at least 2 % by weight of the solid polymer substrate is extracted during the extraction step.

The penetration depth for both the coat polymer and for additional components, if any, may be increased by pretreating the solid polymer substrate with carbon dioxide in supercritical and/or liquid state prior to the application of the polymer coating. The carbon dioxide may e.g. comprise a surfactant for reducing surface tension. In one embodiment, the carbon dioxide in the pretreatment comprises a surfactant preferably selected from the group of anionic, cationic, non-ionic and amphoteric surfactants, said carbon dioxide preferably comprising up to 5% by weight, such as between 0.001-50 grams of surfactant per kg carbon dioxide.

In one embodiment the solid polymer substrate is essentially free of flow tension. Flow tension can be determined by placing the solid polymer substrate in plane-polarized light.

In order to remove or reduce cracks and/or tension in the solid polymer substrate prior to the deposition treatment the solid polymer substrate may in one embodiment be subjected to a pre-heat treatment at a temperature of between T_{g} and T_{g} - 40 °C of the solid polymer substrate, such as between T_{g} and T_{g} - 25 °C of the solid polymer substrate, such as between T_{g} and T_{g} - 15 °C of the solid polymer substrate, such as between T_{g} and T_{g} - 10 °C of the solid polymer substrate.

The pre-heat treatment may e.g. be performed for at least 30 minutes, such as at least 1 hour, such as between 2 and 200 hours, such as between 5 and 100 hours. During the pre-heat treatment an extraction step may simultaneously be performed.

In order to remove or reduce crack formations and/or tension in the solid polymer substrate after deposition treatment the solid polymer substrate may in one embodiment be subjected to a post heat treatment at a temperature of between T_{g} and T_{g} - 40 °C of the coat polymer, such as between T_{g} and T_{g} - 25 °C of the coat polymer, such as between T_{g} and T_{g} - 15 °C of the coat polymer, such as between T_{g} and T_{g} - 10 °C of the coat polymer, the post heat treatment is performed after termination of the deposition step.

The post heat treatment may e.g. be performed for at least 30 minutes, such as at least 1 hour, such as between 2 and 200 hours, such as between 5 and 100 hours.

The dissolution of the coat polymer may be performed at suitable temperatures and pressures. In one embodiment the step of dissolving the coat polymer in a solvent is performed at a temperature of between 10 and 100 °C, such as between 20 and 90 °C, such as between 25 and 80 °C, such as between 30 and 60 °C.

In one embodiment the step of dissolving the coat polymer in a first solvent is performed at a pressure of at least 0.1 MPa, such as between 0.2 and 7.0 MPa, such as below 5 MPa.

The deposition time may vary depending on the desired amount of coating the coat polymer and the condition under the deposition step. In one embodiment the solid polymer substrate is treated with the polymer coat solution in the presence of carbon dioxide in the deposition step for a deposition step time of at least 2 minutes, preferably at least 5, preferably less than 120 minutes, such as between 15 and 90 minutes, such as between 20 and 60 minutes, such as between 25 and 40 minutes. The deposition step time is defined as the point in time where the solid polymer substrate and at least some of the polymer coat solution and at least some carbon dioxide are present in the reaction chamber and the pressure is at least 0.2 MPa, until the pressure in the reactor is reduced to 0.2 MPa or less.

In general it is desired that the deposition time is sufficiently long to apply a continuous and homogenous coating onto the solid polymer substrate.

In one embodiment it is preferred that the carbon dioxide is in its liquid state is subjected to a turbulent movement during at least a part of the deposition step, such as at least 2 minutes, such as at least 5 minutes, such as at least 15 minutes, such as at least 25 minutes. The carbon dioxide and the solid polymer substrate may thus preferably be subjected to a mechanical mixing during at least a part of the deposition step, such as at least 2 minutes, such as at least 5 minutes, such as at least 15 minutes, such as at least 25 minutes.

It has thus been found that the solvability of the active compound in the liquid carbon dioxide can be increased by subjecting it to turbulent movements such as stirring or mixing. In one embodiment the mixing can be obtained by a tumbling system or a stirrer.

A tumbler might rotate with 7 -60 rpm. There might be fixed wings inside the tumbler to obtain a better mixing between liquid and the items.
A stirrer might be formed as wings and might rotate with 10 - 500 rpm.

In order to reduce deposition time, particularly if the pressure during the deposition step is less than 3.0 MPa, the carbon dioxide may e.g. comprise a surfactant for reducing surface tension. In one embodiment, the carbon dioxide in the pretreatment comprises a surfactant preferably selected from the group of anionic, cationic, non-ionic and amphoteric surfactants, said carbon dioxide preferably comprising up to 5% by weight, such as between 0.001-50 grams of surfactant per kg carbon dioxide.

In one embodiment the carbon dioxide during the deposition step is in its liquid state for at least 1 minute, preferably the carbon dioxide is in its liquid state for at least 2 minutes, such as at least 5 minutes, such as at least 15 minutes, such as at least 25 minutes during the deposition step.

In one embodiment, the pressure and/or the temperature may be varied during the deposition step e.g. pulsed. The pressure in the reactor during the deposition step may thus be pulsed with one or more pulse, wherein one pulse includes raising the pressure by at least 0.1 MPa, followed by lowering the pressure by at least 0.1 MPa, followed by increasing the pressure by at least 0.1 MPa.

In one embodiment the pressure variation during at least a part of the deposition step time is kept within 1.0 MPa /minute in order to avoid damaging the product. This method is in particular useful for glassy solid polymer substrate, such as polystyrene and polycarbonate. In one embodiment the pressure variation during at least 10 %, such as at least 50 %, such as at least 90 % of the deposition step time is less than 1.0 MPa /minute, such as less than 0.5 MPa /minute, such as less than 0.2 MPa /minute, such as less than 0.1 MPa /minute.

In one embodiment the pressure reduction half-life (t_{½P}) during at least 50 % of the deposition step time is substantially constant. The term "constant" means in this connection within +- 10 % from the average reduction half-life.

The temperature and the pressure may be regulated so that the carbon dioxide is changing state during the deposition step.

In one embodiment the temperature during the deposition step is at least 0 °C, such as between 5 and 100 °C, such as between 5 and 15 °C.

In one embodiment the pressure in the reactor during at least 10 %, such as at least 50 %, such as at least 70 % of the deposition step time, such as between 50 and 90 % of the deposition step time being above 0.5 MPa, preferably between 1.0 and 7.4 MPa, such as between 1.2 and 7.0 MPa. In this embodiment the carbon dioxide may preferably be in its liquid state.

In one embodiment the pressure in the reactor during at least 10 %, such as at least 50 %, such as at least 70 % of the deposition step time, such as between 50 and 90 % of the deposition step time being between 0.5 and 3.0 MPa, such as between 1.0 and 2.0 MPa. In this embodiment the carbon dioxide may preferably be in its liquid state.

Due to the cost of equipment it is preferred to carry out the deposition at a pressure of 5.5 MPa or less, such as 3.0 MPa or less, such as 2.0 MPa or less.

In order to avoid damaging the material during decompression, the decompression should preferably be performed relatively slowly. In one embodiment the pressure reduction at the last 10 % of the deposition step time is less than 2.0 MPa /minute, such as less than 1.5 MPa /minute, such as less than 1.0 MPa /minute, such as less than 0.5 MPa /minute, such as less than 0.2 MPa /minute, such as less than 0.1 MPa /minute.

In one embodiment the pressure in the reactor is reduced from 2 to 1 bar as slowly as or even slower than the reduction from 3 to 2 bars.

The deposited coatings may preferably have a thickness of between 10⁻⁵ µg/cm² and 1 mg/cm², such as between 10⁻⁴ µg/cm² and 10 µg/cm², such as between 10⁻³ µg/cm² and 1 µg/cm². The desired thickness depends on the application of the product. The thickness may vary or preferably be evenly distributed over the treated surface of the solid polymer substrate.

The invention also relates to an item obtainable by the method.

Preferred items include a medical device such as a medical device for use in contact with the human body, e.g. a contact lens, a catheter, an implant (e.g. a synthetic blood vein, a hip implant, a sinus-shunts (curing of hydrocephalus), prosthesis like guide wires, and other polymer containing implants) and a contact lens.

Other preferred items include laboratory utensils, such as test tube/plate e.g. an Elisa plate, a flow cell, a slide, and a stirrer.

### EXAMPLE

20 polystyrene Elisa plates produced by injecting molding are pretreated by heating in an oven at 90 °C for four days for removing flow tension. The 20 polystyrene plates are placed in a stacker which is placed in the reaction vessel to ensure sufficient stirring during the process. A standard 0.50% wt MPC in ethanol solution is produced, by stirring 20.00 g of MPC and 5.00 L Ethanol over night. After placing the polystyrene plates in the reaction vessel the reaction vessel is closed and CO₂ gas is added to a pressure of 5.10 MPa at 15 °C. Then approx. 90 L 1.66 % wt MPC/EtOH solution in liquid CO₂ is added to the reaction vessel. The pressure is held at 5.10 MPa at 15 °C for 15 minutes where after the decompression occurs at a constant velocity over 15 min.

## Claims

1. A method of coating a surface of a solid polymer substrate with a coating comprising a coat polymer, said method comprises the steps of
a) providing a coat polymer for the coating, and dissolving said coat polymer in a first solvent comprising at least 50 % by weight of an organic solvent,
b) placing said solid polymer substrate in a reaction chamber
c) introducing said polymer coat solution into the reaction chamber,
d) introducing carbon dioxide into the reaction chamber, and
e) depositing the coating onto the solid polymer substrate surface,
wherein the carbon dioxide during at least a part of the deposition step is in its liquid state at a pressure up to 7.4 MPa; the polymer substrate is selected from the group consisting of silicone polymers, thermoplastic elastomers, rubbers, polyolefins, polyesters, polystyrene, polyacrylates, polyethers, polyurethane, polycarbonate, thermoplastic vulcanisates, epoxy polymers, thermoset polyimide and mixtures thereof, and the coat polymer comprises one or more of the polymers selected from the group consisting of PTFE, phospholipid containing polymers; butyl metacrylate containing polymers (PBMA); poly(2-hydroxyethyl metacrylate) (PHEMA) containing polymers; polydimethylsiloxane (PDMS); poly(ethylene glycol) (PEG); vinylpyrrolidone containing polymer and copolymers thereof.

2. A method according claim 1 wherein the solid polymer substrate is of a polymer composition comprising at least 10 % by weight of a thermoplastic elastomer selected from the group consisting of a random copolymer, and a block copolymer.

3. A method according to any one of the preceding claims wherein the solid polymer substrate is of a polymer composition comprising at least 10 % by weight of a rubber selected from the group consisting of butadiene rubber, isoprene rubber, nitril rubber, styrene-butadiene rubber, latex and urethane rubber.

4. A method according to any one of the preceding claims wherein the solid polymer substrate is of a polymer composition comprising at least 10 % by weight of a polyolefin selected from the group consisting of polyvinyls.

5. A method according to any one of the preceding claims wherein the solid polymer substrate is of a polymer composition comprising at least 10 % by weight of silicone polymers selected from the group consisting of dimethyl polysiloxane, methylphenyl polysiloxane, fluorosilicone rubber, silicone esters, polysiloxanes, polysilanes, chlorosilanes, alkoxysilanes, aminosilanes, polysilanes polydialkylsiloxanes, polysiloxanes containing phenyl substituents, said polymers of the silicone polymer composition optionally being vinyl-functionalized and/or optionally being partially or fully fluorinated.

6. A method according to any one of the preceding claims wherein the solid polymer substrate is of a polymer composition comprising at up to 40 % by weight of fillers and/or additives.

7. A method according to any one of the preceding claims wherein the coat polymer comprises a polymer which is more oleophobic than the solid polymer substrate.

8. A method according to any one of the preceding claims wherein the coat polymer comprises a polymer which is more hydrophilic than the solid polymer substrate.

9. A method according to any one of the preceding claims wherein the coat polymer comprises one or more polymers with anti oxidizing and/or electrical conductive properties.

10. A method according to any one of the preceding claims wherein the coat polymer has an average number molecular weight of at least 5,000.

11. A method according to any one of the preceding claims wherein the coat polymer comprises one or more of the polymers 2-methacryloyloxyethylphosphorylcholine (MPC) containing polymers and poly(vinylpyrrolidone) (PVP).

12. A method according to any one of the preceding claims wherein the coat polymer comprises one or more polymer having metacrylate backbone.

13. A method according to any one of the preceding claims wherein said first solvent comprises one or more of water, carbon dioxide, cyclohexanones; alcohols; acids of alcohols, esters and toluene.

14. A method according to any one of the preceding claims wherein said first solvent comprises at least 75 % by weight of an organic solvent.

15. A method according to any one of the preceding claims wherein said first solvent comprises a mixture of water and one or more organic solvents.

16. A method according to any one of the preceding claims wherein said first solvent comprises a mixture of carbon dioxide and one or more organic solvents.

17. A method according to any one of the preceding claims wherein the method further includes dissolving or dispersing at least one additional component in the first solvent.

18. A method according to claim 17 wherein the additional component being selected from the group consisting of pigments; anti-thrombotic agents,; anti-microbial agents and antioxidants.

19. A method according to any one of the preceding claims wherein the solid polymer substrate is placed in the reaction chamber, carbon dioxide is introduced into the reaction chamber to raise the pressure to at least 0.2 MPa, where after the polymer coat solution is injected into the reaction chamber.

20. A method according to claim 19 wherein the pressure within the reaction chamber is raised to at least 1.5 MPa prior to injecting the polymer coat solution into the reaction chamber.

21. A method according to claim 19 wherein the reaction chamber at the time of introducing the polymer coat solution into the reaction chamber comprises carbon dioxide in its liquid and state.

22. A method according to any one of the preceding claims wherein the polymer coat solution is injected into the reaction chamber together with carbon dioxide.

23. A method according to any one of the preceding claims comprising the steps of dissolving the coat polymer in a first solvent, followed by mixing said polymer coat solution with carbon dioxide in gas form where after the polymer coat solution carbon dioxide mixture is injected into the reaction chamber.

24. A method according to any one of the preceding claims 1-22 comprising the steps of dissolving the coat polymer in a first solvent, followed by mixing said polymer coat solution with carbon dioxide in liquid form where after the polymer coat solution carbon dioxide mixture being is injected into the reaction chamber.

25. A method according to any one of the preceding claims wherein the step of dissolving the coat polymer in a first solvent is performed at a temperature of between 10 and 100 °C.

26. A method according to any one of the preceding claims wherein the step of dissolving the coat polymer in a first solvent is performed at a pressure of at least 0.1 and below 5 MPa.

27. A method according to any one of the preceding claims wherein the step of dissolving the coat polymer in a first solvent is performed prior to its introduction into the reactor chamber.

28. A method according to any one of the preceding claims wherein the step of dissolving the coat polymer in a first solvent is performed prior to bringing it into contact with the solid polymer substrate.

29. A method according to any one of the preceding claims wherein the solid polymer substrate is treated with the polymer coat solution in the presence of carbon dioxide in the deposition step for a deposition step time of at least 2 minutes and less than 120 minutes, the deposition step time being defined as the point in time where the solid polymer substrate and at least some of the polymer coat solution and at least some carbon dioxide are present in the reaction chamber and the pressure is at least 0.2 MPa until the pressure in the reactor is reduced to 0.2 MPa or less.

30. A method according to any one of the preceding claims wherein the deposition time is sufficiently long to apply a continuous coating onto the solid polymer substrate.

31. A method according to any one of the preceding claims wherein the carbon dioxide for at least 1 minute during the deposition step is in its liquid state.

32. A method according to any one of the preceding claims wherein the carbon dioxide is in its liquid state is subjected to a turbulent movement during at least a part of the deposition step.

33. A method according to any one of the preceding claims wherein the temperature during the deposition step is between 5 and 100 °C.

34. A method according to any one of the preceding claims wherein the pressure in the reactor during at least 10 % of the deposition step time is above 0.5 MPa.

35. A method according to any one of the preceding claims wherein the pressure in the reactor during at least 10 % of the deposition step time is between 0.5 and 3.0 MPa.

36. A method according to any one of the preceding claims wherein the pressure in the reactor during the deposition step is be pulsed with one or more pulses, wherein one pulse includes raising the pressure by at least 0.1 MPa, followed by lowering the pressure by at least 0.1 MPa followed by increasing the pressure by at least 0.1 MPa.

37. A method according to any one of the preceding claims wherein the pressure variation during at least 10 % of the deposition step time is less than 1.0 MPa /minute.

38. A method according to any one of the preceding claims wherein the pressure reduction half-life (t_{½P}) during at least 50 % of the deposition step time is substantially constant.

39. A method according to any one of the preceding claims wherein the pressure reduction at the last 10 % of the deposition step time is less than 2.0 MPa /minute.

40. A method according to any one of the preceding claims wherein the pressure in the reactor is reduced from 2 to 1 bar as slowly as or slower than the reduction from 3 to 2 bars.

41. A method according to any one of the preceding claims wherein the solid polymer substrate is subjected to a pretreatment prior to the step of applying the coating, the pretreatment comprises an extraction step, the extraction step includes extraction of oils and water from the solid polymer substrate.

42. A method according to claim 41 wherein the extraction step is performed by subjecting the solid polymer substrate to one or more of the treatments, a heat treatment, a vacuum treatment, a treatment with a supercritical solvent and a treatment with liquid carbon dioxide.

43. A method according to any one of the claims 41 and 42 wherein at least 0.05% by weight of the solid polymer substrate is extracted during the extraction step.

44. A method according to any one of the preceding claims wherein the solid polymer substrate is subjected to a post heat treatment at a temperature of between T_{g} and T_{g} - 40 °C of the coat polymer, the post heat treatment being performed after termination of the deposition step.

45. A method according to claim 44 wherein the post heat treatment is performed for at least 30 minutes.

46. A method according to any one of the preceding claims wherein the deposited coating has a thickness of between 10⁻⁵ µg/cm² and 1 mg/cm².

47. A method according to any one of the preceding claims wherein the deposited coating has an essentially even thickness over the treated surface of the solid polymer substrate.

48. An item obtainable using the method as defined in any one of the claims 1-47.

49. An item according to claim 48, wherein the item is a medical device for use in contact with the human body.

50. An item according to claim 48, wherein the item is a laboratory utensil.

## Patentansprüche

1. Verfahren zur Beschichtung einer Oberfläche eines festen Polymersubstrats mit einer Beschichtung umfassend ein Schichtpolymer, wobei das Verfahren die folgenden Stufen umfasst, dass
a) ein Schichtpolymer für die Beschichtung bereitgestellt wird und das Schichtpolymer in einem ersten Lösungsmittel gelöst wird, das mindestens 50 Gew.-% eines organischen Lösungsmittels enthält,
b) das feste Polymersubstrat in eine Reaktionskammer gebracht wird,
c) die Schichtpolymerlösung in die Reaktionskammer gebracht wird,
d) Kohlendioxid in die Reaktionskammer geleitet wird und
e) die Beschichtung auf der festen Polymersubstratoberfläche abgeschieden wird,
wobei das Kohlendioxid mindestens während eines Teils der Abscheidungsstufe in flüssigem Zustand ist mit einem Druck von bis zu 7,4 MPa; das Polymersubstrat ausgewählt ist aus der Gruppe bestehend aus Siliconpolymeren, thermoplastischen Elastomeren, Kautschuken, Polyolefinen, Polyestern, Polystyrol, Polyacrylaten, Polyethern, Polyurethan, Polycarbonat, thermoplastischen Vulkanisaten, Epoxypolymeren, hitzehärtbarem Polyimid und Mischungen davon und das Schichtpolymer ein oder mehrere der Polymere aufweist ausgewählt aus der Gruppe bestehend aus PTFE, phospholipidhaltigen Polymeren; Butylmethacrylat enthaltenden Polymeren (PBMA); Poly-(2-hydroxyethylmethacrylat)-(PHEMA)-haltigen Polymeren; Polydimethylsiloxan (PDMS); Poly(ethylenglycol) (PEG); vinylpyrrolidonhaltigem Polymer und Copolymeren davon.

2. Verfahren nach Anspruch 1, wobei das feste Polymersubstrat aus einer Polymerzusammensetzung ist, die mindestens 10 Gew.-% eines thermoplastischen Elastomers ausgewählt aus der Gruppe bestehend aus einem statistischen Copolymer und einem Blockcopolymer enthält.

3. Verfahren nach einem der vorhergehenden Ansprüche, wobei das feste Polymersubstrat aus einer Polymerzusammensetzung ist, die mindestens 10 Gew.-% eines Kautschuks enthält ausgewählt aus der Gruppe bestehend aus Butadienkautschuk, Isoprenkautschuk, Nitrilkautschuk, Styrolbutadienkautschuk, Latex und Urethankautschuk.

4. Verfahren nach einem der vorhergehenden Ansprüche, wobei das feste Polymersubstrat aus einer Polymerzusammensetzung ist, die mindestens 10 Gew.-% eines Polyolefins enthält ausgewählt aus der Gruppe bestehend aus Polyvinylen.

5. Verfahren nach einem der vorhergehenden Ansprüche, wobei das feste Polymersubstrat aus einer Polymerzusammensetzung ist, die mindestens 10 Gew.-% Siliconpolymere enthält ausgewählt aus der Gruppe bestehend aus Dimethylpolysiloxan, Methylphenylpolysiloxan, Fluorsiliconkautschuk, Siliconestern, Polysiloxanen, Polysilanen, Chlorsilanen, Alkoxysilanen, Aminosilanen, Polysilanen, Polydialkylsiloxanen, Polysiloxanen, die Phenylsubstituenten enthalten, wobei die Polymere der Siliconpolymerzusammensetzung gegebenenfalls vinylfunktionalisiert und/oder gegebenenfalls teilweise oder vollständig fluoriert sind.

6. Verfahren nach einem der vorhergehenden Ansprüche, wobei das feste Polymersubstrat aus einer Polymerzusammensetzung ist, die bis zu 40 Gew.-% Füllstoffe und/oder Zusatzstoffe enthält.

7. Verfahren nach einem der vorhergehenden Ansprüche, wobei das Schichtpolymer ein Polymer enthält, das oleophober ist als das feste Polymersubstrat.

8. Verfahren nach einem der vorhergehenden Ansprüche, wobei das Schichtpolymer ein Polymer enthält, das hydrophiler ist als das feste Polymersubstrat.

9. Verfahren nach einem der vorhergehenden Ansprüche, wobei das Schichtpolymer ein oder mehrere Polymere mit antioxidierenden und/oder elektrisch leitenden Eigenschaften enthält.

10. Verfahren nach einem der vorhergehenden Ansprüche, wobei das Schichtpolymer ein zahlenmittleres Molekulargewicht von mindestens 5.000 hat.

11. Verfahren nach einem der vorhergehenden Ansprüche, wobei das Schichtpolymer ein oder mehrere der Polymere 2-Methacryloyloxyethylphosphorylcholin (MPC)-haltige Polymere und Poly(vinylpyrrolidon) (PVP) enthält.

12. Verfahren nach einem der vorhergehenden Ansprüche, wobei das Schichtpolymer ein oder mehrere Polymere mit Methacrylatgerüst enthält.

13. Verfahren nach einem der vorhergehenden Ansprüche, wobei das erste Lösungsmittel ein oder mehere der Lösungsmittel Wasser, Kohlendioxid, Cyclohexanone, Alkohol, Säuren von Alkoholen, Ester und Toluol enthält.

14. Verfahren nach einem der vorhergehenden Ansprüche, wobei das erste Lösungsmittel mindestens 75 Gew.-% eines organischen Lösungsmittels enthält.

15. Verfahren nach einem der vorhergehenden Ansprüche, wobei das erste Lösungsmittel eine Mischung aus Wasser und einem oder mehreren organischen Lösungsmitteln enthält.

16. Verfahren nach einem der vorhergehenden Ansprüche, wobei das erste Lösungsmittel eine Mischung aus Kohlendioxid und einem oder mehreren organischen Lösungsmitteln enthält.

17. Verfahren nach einem der vorhergehenden Ansprüche, wobei das Verfahren weiterhin beinhaltet, dass mindestens eine zusätzliche Komponente in dem ersten Lösungsmittel gelöst oder dispergiert wird.

18. Verfahren nach Anspruch 17, wobei die zusätzliche Komponente ausgewählt ist aus der Gruppe bestehend aus Pigmenten; gerinnungshemmenden Mitteln; antimikrobiellen Mitteln und Antioxidantien.

19. Verfahren nach einem der vorhergehenden Ansprüche, wobei das feste Polymersubstrat in die Reaktionskammer gebracht wird, Kohlendioxid in die Reaktionskammer eingeleitet wird, um den Druck auf mindestens 0,2 MPa zu erhöhen, wonach die Polymerschichtlösung in die Reaktionskammer injiziert wird.

20. Verfahren nach Anspruch 19, wobei der Druck in der Reaktionskammer vor dem Injizieren der Schichtpolymerlösung in die Reaktionskammer auf mindestens 1,5 MPa erhöht wird.

21. Verfahren nach Anspruch 19, wobei die Reaktionskammer zum Zeitpunkt des Einleitens der Schichtpolymerlösung in die Reaktionskammer Kohlendioxid in flüssigem Zustand enthält.

22. Verfahren nach einem der vorhergehenden Anspruche, wobei die Schichtpolymerlösung in die Reaktionskammer zusammen mit dem Kohlendioxid injiziert wird.

23. Verfahren nach einem der vorhergehenden Anspruche, das die Stufen aufweist, dass das Schichtpolymer in einem ersten Lösungsmittel gelöst wird, anschließend die Schichtpolymerlösung mit Kohlendioxid in Gasform vermischt wird, wonach die Polymerschichtlösung-Kohlendioxid-Mischung in die Reaktionskammer injiziert wird.

24. Verfahren nach einem der vorhergehenden Ansprüche 1 bis 22, das die Stufen umfasst, dass das Schichtpolymer in einem ersten Lösungsmittel gelöst wird, anschließend die Schichtpolymerlösung mit Kohlendioxid in flüssiger Form vermischt wird, wonach die Schichtpolymerlösung-Kohlendioxid-Mischung in die Reaktionskammer injiziert wird.

25. Verfahren nach einem der vorhergehenden Ansprüche, wobei die Stufe, bei der das Schichtpolymer in einem ersten Lösungsmittel gelöst wird, bei einer Temperatur zwischen 10 und 100°C durchgeführt wird.

26. Verfahren nach einem der vorhergehenden Ansprüche, wobei die Stufe, bei der das Schichtpolymer in einem ersten Lösungsmittel gelöst wird, bei einem Druck von mindestens 0,1 und weniger als 5 MPa durchgeführt wird.

27. Verfahren nach einem der vorhergehenden Ansprüche, wobei die Stufe, bei der das Schichtpolymer in einem ersten Lösungsmittel gelöst wird, durchgerührt wird, bevor es in die Reaktionskammer eingeleitet wird.

28. Verfahren nach einem der vorhergehenden Ansprüche, wobei die Stufe des Auflösens des Schichtpolymers in einem ersten Lösungsmittel durchgeführt wird, bevor es mit dem festen Polymersubstrat in Kontakt gebracht wird.

29. Verfahren nach einem der vorhergehenden Ansprüche, wobei das feste Polymersubstrat mit der Polymerschichtlösung in Gegenwart von Kohlendioxid in der Abscheidungsstufe für eine Abscheidungszeit von mindestens 2 Minuten und weniger als 120 Minuten behandelt wird, wobei die Zeit für die Abscheidungsstufe definiert wird als der Zeitpunkt, zu dem das feste Polymersubstrat und mindestens etwas von der Polymerschichtlösung und mindestens etwas von dem Kohlendioxid in der Reaktionskammer vorhanden sind und der Druck mindestens 0,2 MPa ist, bis der Druck in dem Reaktor auf 0,2 MPa oder weniger reduziert wird.

30. Verfahren nach einem der vorhergehenden Ansprüche, wobei der Abscheidungszeitraum ausreichend lang ist, um eine kontinuierliche Beschichtung auf das feste Polymersubstrat aufzubringen.

31. Verfahren nach einem der vorhergehenden Anspruche, wobei das Kohlendioxid mindestens 1 Minute während der Abscheidungsstufe in flüssigem Zustand ist

32. Verfahren nach einem der vorhergehenden Ansprüche, wobei das Kohlendioxid in flüssigem Zustand einer turbulenten Bewegung während mindestens eines Teils der Abscheidungsstufe unterzogen wird.

33. Verfahren nach einem der vorhergehenden Ansprüche, wobei die Temperatur während der Abscheidungsstufe zwischen 5 und 100°C liegt.

34. Verfahren nach einem der vorhergehenden Ansprüche, wobei der Druck in dem Reaktor mindestens während 10% der Abscheidungszeit über 0,5 MPa ist.

35. Verfahren nach einem der vorhergehenden Ansprüche, wobei der Druck in dem Reaktor während mindestens 10% der Abscheidungszeit zwischen 0,5 und 3,0 MPa liegt.

36. Verfahren nach einem der vorhergehenden Ansprüche, wobei der Druck in dem Reaktor während der Abscheidungsstufe mit einem oder mehreren Pulsen gepulst wird, wobei ein Puls einschließt, dass der Druck um mindestens 0,1 MPa erhöht wird, anschließend der Druck um mindestens 0,1 MPa gesenkt wird, anschließend der Druck um mindestens 0,1 MPa erhöht wird.

37. Verfahren nach einem der vorhergehenden Ansprüche, wobei die Druckvariation während mindestens 10% der Abscheidungszeit geringer als 1,0 MPa/min ist.

38. Verfahren nach einem der vorhergehenden Ansprüche, wobei die Druckreduktionshalbwertszeit (t_{½p}) während mindestens 50% der Abscheidungszeit im Wesentlichen konstant ist.

39. Verfahren nach einem der vorhergehenden Ansprüche, wobei die Druckreduktion bei den letzten 10% der Abscheidungszeit geringer als 2,0 MPa/min ist.

40. Verfahren nach einem der vorhergehenden Ansprüche, wobei der Druck in dem Reaktor von 2 auf 1 bar so langsam wie oder langsamer reduziert wird, als die Reduktion von 3 auf 2 bar.

41. Verfahren nach einem der vorhergehenden Ansprüche, wobei das feste Polymersubstrat einer Vorbehandlung unterzogen wird vor der Stufe, bei der die Beschichtung aufgebracht wird, wobei die Vorbehandlung eine Extraktionsstufe umfasst, die Extraktionsstufe die Extraktion von Ölen und Wasser aus dem festen Polymersubstrat einschließt.

42. Verfahren nach Anspruch 41, wobei die Extraktionsstufe durchgeführt wird, indem das feste Polymersubstrat einer Wärmebehandlung und/oder einer Vakuumbehandlung und/oder einer Behandlung mit überkritischem Lösungsmittel und/oder einer Behandlung mit flüssigem Kohlendioxid unterzogen wird.

43. Verfahren nach einem der Ansprüche 41 und 42, wobei mindestens 0,05 Gew.-% des festen Polymersubstrats während der Extraktionsstufe extrahiert werden.

44. Verfahren nach einem der vorhergehenden Ansprüche, wobei das feste Polymersubstrat einer Nachwärmebehandlung bei einer Temperatur zwischen T_{g} und T_{g} - 40°C des Schichtpolymers unterzogen wird, wobei die Nachwärmebehandlung nach dem Ende der Abscheidungsstufe durchgeführt wird.

45. Verfahren nach Anspruch 44, wobei die Nachwärmebehandlung mindestens 30 Minuten lang durchgeführt wird.

46. Verfahren nach einem der vorhergehenden Ansprüche, wobei die Abscheidungsbeschichtung eine Dicke zwischen 10⁻⁵ µg/cm² und 1 mg/cm² hat.

47. Verfahren nach einem der vorhergehenden Anspruch, wobei die Dicke der abgeschiedenen Beschichtung über die behandelte Oberfläche des festen Polymersubstrats im Wesentlichen gleichmäßig ist.

48. Gegenstand erhältlich unter Verwendung des Verfahrens, wie es in einem der Ansprüche 1 bis 47 definiert ist.

49. Gegenstand nach Anspruch 48, wobei der Gegenstand eine medizinische Vorrichtung zur Verwendung in Kontakt mit dem menschlichen Körper ist.

50. Gegenstand nach Anspruch 48, wobei der Gegenstand ein Laborutensil ist.

## Revendications

1. Procédé de revêtement d'une surface d'un substrat polymère solide avec un revêtement comprenant un polymère de revêtement, ledit procédé comprenant les étapes consistant à :
a) fournir un polymère de revêtement pour le revêtement, et dissoudre ledit polymère de revêtement dans un premier solvant comprenant au moins 50 % en poids d'un solvant organique,
b) placer ledit substrat polymère solide dans une chambre de réaction,
c) introduire ladite solution de revêtement de polymère dans la chambre de réaction,
d) introduire du dioxyde de carbone dans la chambre de réaction, et
e) déposer le revêtement sur la surface du substrat polymère solide,
dans lequel le dioxyde de carbone, pendant au moins une partie de l'étape de dépôt, se trouve dans son état liquide à une pression pouvant atteindre 7,4 MPa; le substrat polymère est sélectionné dans le groupe comprenant des polymères de silicone, des élastomères thermoplastiques, des caoutchoucs, des polyoléfines, des polyesters, du polystyrène, des polyacrylates, des polyéthers, du polyuréthane, du polycarbonate, des vulcanisats thermoplastiques, des polymères d'époxy, du polyimide thermodurci et des mélanges de ceux-ci, et le polymère d'enrobage comprend un ou plusieurs des polymères sélectionnés dans le groupe comprenant le PTFE, des polymères contenant des phospholipides, des polymères contenant du butyle méthacrylate (PBMA), des polymères contenant du poly(2-hydroxyéthyle méthacrylate) (PHEMA), du polydiméthylsiloxane (PDMS), du poly-(éthylène glycol) (PEG); un polymère contenant du vinyle pyrrolidone, et des copolymères de ceux-ci.

2. Procédé selon la revendication 1, dans lequel le substrat polymère solide est constitué d'une composition polymère qui comprend au moins 10 % en poids d'un élastomère thermoplastique sélectionné dans le groupe comprenant un copolymère aléatoire et un copolymère bloc.

3. Procédé selon l'une quelconque des revendications précédentes, dans lequel le substrat polymère solide est constitué d'une composition polymère comprenant au moins 10 % en poids d'un caoutchouc sélectionné dans le groupe comprenant le caoutchouc butadiène, le caoutchouc isoprène, le caoutchouc nitrile, le caoutchouc styrène-butadiène, le latex et le caoutchouc uréthane.

4. Procédé selon l'une quelconque des revendications précédentes, dans lequel le substrat polymère solide est constitué d'une composition polymère comprenant au moins 10 % en poids d'une polyoléfine sélectionnée dans le groupe consistant en des polyvinyles.

5. Procédé selon l'une quelconque des revendications précédentes, dans lequel le substrat polymère solide est constitué d'une composition polymère comprenant au moins 10 % en poids de polymères de silicone sélectionnés dans le groupe consistant en le diméthyle polysiloxane, le méthylphényle polysiloxane, le caoutchouc de fluorosilicone, des esters de silicone, des polysiloxanes, des polysilanes, des chlorosilanes, des alkoxysilanes, des aminosilanes, des polysilanes, des polydialkylsiloxanes, des polysiloxanes contenant des substituants de phényle, lesdits polymères de la composition polymère de silicone étant optionnellement fonctionnalisés au vinyle et/ou étant optionnellement partiellement ou complètement fluorés.

6. Procédé selon l'une quelconque des revendications précédentes, dans lequel le substrat polymère solide est constitué d'une composition polymère qui contient jusqu'à 40 % en poids d'agents de remplissage et/ou d'additifs.

7. Procédé selon l'une quelconque des revendications précédentes, dans lequel le polymère de revêtement comprend un polymère qui est plus oléophobe que le substrat polymère solide.

8. Procédé selon l'une quelconque des revendications précédentes, dans lequel le polymère de revêtement comprend un polymère qui est plus hydrophile que le substrat polymère solide.

9. Procédé selon l'une quelconque des revendications précédentes, dans lequel le polymère de revêtement comprend un ou plusieurs polymère(s) qui présente(nt) des propriétés anti-oxydantes et/ou de conduction d'électricité.

10. Procédé selon l'une quelconque des revendications précédentes, dans lequel le polymère de revêtement présente un poids moléculaire moyen d'au moins 5 000.

11. Procédé selon l'une quelconque des revendications précédentes, dans lequel le polymère d'enrobage comprend un ou plusieurs des polymères contenant du 2-méthacryloyloxyéthylphosphorylcholine (MPC) et du poly(vinylpyrrolidone) (PVP).

12. Procédé selon l'une quelconque des revendications précédentes, dans lequel le polymère d'enrobage comprend un ou plusieurs polymère(s) possédant une ossature de méthacrylate.

13. Procédé selon l'une quelconque des revendications précédentes, dans lequel ledit premier solvant comprend un ou plusieurs éléments parmi l'eau, le dioxyde de carbone, des cyclohexanones, des alcools, des acides d'alcools, des esters et le toluène.

14. Procédé selon l'une quelconque des revendications précédentes, dans lequel ledit premier solvant comprend au moins 75 % en poids d'un solvant organique.

15. Procédé selon l'une quelconque des revendications précédentes, dans lequel ledit premier solvant comprend un mélange d'eau et d'un ou de plusieurs solvant(s) organique(s).

16. Procédé selon l'une quelconque des revendications précédentes, dans lequel ledit premier solvant comprend un mélange de dioxyde de carbone et d'un ou de plusieurs solvant(s) organique(s).

17. Procédé selon l'une quelconque des revendications précédentes, dans lequel le procédé comprend en outre la dissolution ou la dispersion d'un composant supplémentaire dans le premier solvant.

18. Procédé selon la revendication 17, dans lequel le composant supplémentaire est sélectionné dans le groupe comprenant des pigments, des agents anti-thrombotiques, des agents anti-microbiens et des antioxydants.

19. Procédé selon l'une quelconque des revendications précédentes, dans lequel le substrat polymère solide est placé dans la chambre de réaction, du dioxyde de carbone est introduit dans la chambre de réaction pour faire monter la pression jusqu'à au moins 0,2 MPa, et ensuite la solution d'enrobage de polymère est injectée dans la chambre de réaction.

20. Procédé selon la revendication 19, dans lequel la pression à l'intérieur de la chambre de réaction est augmentée jusqu'à au moins 1,5 MPa avant d'injecter la solution de revêtement de polymère dans la chambre de réaction.

21. Procédé selon la revendication 19, dans lequel la chambre de réaction au moment de l'introduction de la solution de revêtement de polymère dans la chambre de réaction comprend du dioxyde de carbone dans son état liquide.

22. Procédé selon l'une quelconque des revendications précédentes, dans lequel la solution de revêtement de polymère est injectée dans la chambre de réaction simultanément avec le dioxyde de carbone.

23. Procédé selon l'une quelconque des revendications précédentes, comprenant les étapes de dissolution du polymère de revêtement dans un premier solvant, suivie par le mélange de ladite solution de revêtement de polymère avec du dioxyde de carbone à l'état gazeux, et ensuite le mélange de solution de revêtement de polymère et de dioxyde de carbone est injecté dans la chambre de réaction.

24. Procédé selon l'une quelconque des revendications précédentes 1 à 22, comprenant les étapes de dissolution du polymère de revêtement dans un premier solvant, suivie par le mélange de ladite solution de revêtement de polymère avec du dioxyde de carbone à l'état liquide, et ensuite le mélange de solution de revêtement de polymère et de dioxyde de carbone est injecté dans la chambre de réaction.

25. Procédé selon l'une quelconque des revendications précédentes, dans lequel l'étape de dissolution du polymère de revêtement dans un premier solvant est exécutée à une température qui est comprise entre 10°C et 100°C.

26. Procédé selon l'une quelconque des revendications précédentes, dans lequel l'étape de dissolution du polymère de revêtement dans un premier solvant est exécutée à une pression d'au moins 0,1 MPa et inférieure à 5 MPa.

27. Procédé selon l'une quelconque des revendications précédentes, dans lequel l'étape de dissolution du polymère de revêtement dans un premier solvant est exécutée avant son introduction dans la chambre de réaction.

28. Procédé selon l'une quelconque des revendications précédentes, dans lequel l'étape de dissolution du polymère de revêtement dans un premier solvant est exécutée avant de l'amener en contact avec le substrat polymère solide.

29. Procédé selon l'une quelconque des revendications précédentes, dans lequel le substrat polymère solide est traité avec la solution de revêtement de polymère en présence de dioxyde de carbone pendant l'étape de dépôt pour une durée de l'étape de dépôt d'au moins 2 minutes, et inférieure à 120 minutes, la durée de l'étape de dépôt étant définie comme étant le moment pendant lequel le substrat polymère solide et au moins une partie de la solution de revêtement de polymère et au moins une partie du dioxyde de carbone sont présents dans la chambre de réaction et pendant lequel la pression est au moins de 0,2 MPa jusqu'à ce que la pression dans le réacteur soit ramenée à 0,2 MPa, ou moins.

30. Procédé selon l'une quelconque des revendications précédentes, dans lequel la durée du dépôt est suffisamment longue pour appliquer un revêtement continu sur le substrat polymère solide.

31. Procédé selon l'une quelconque des revendications précédentes, dans lequel le dioxyde de carbone, pendant au moins 1 minute pendant l'étape de dépôt, se trouve dans son état liquide.

32. Procédé selon l'une quelconque des revendications précédentes, dans lequel le dioxyde de carbone, lorsqu'il se trouve dans son état liquide, est soumis à un mouvement turbulent pendant au moins une partie de l'étape de dépôt.

33. Procédé selon l'une quelconque des revendications précédentes, dans lequel la température pendant l'étape de dépôt est comprise entre 5°C et 100°C.

34. Procédé selon l'une quelconque des revendications précédentes, dans lequel la pression dans le réacteur pendant au moins 10 % de la durée de l'étape de dépôt est supérieure à 0,5 MPa.

35. Procédé selon l'une quelconque des revendications précédentes, dans lequel la pression dans le réacteur pendant au moins 10 % de la durée de l'étape de dépôt est comprise entre 0,5 MPa et 3,0 MPa.

36. Procédé selon l'une quelconque des revendications précédentes, dans lequel la pression dans le réacteur pendant l'étape de dépôt doit être pulsée avec une ou plusieurs impulsion(s), dans lequel une impulsion comprend l'augmentation de la pression d'au moins 0,1 MPa, suivie par une diminution de la pression d'au moins 0,1 MPa, suivie par une augmentation de la pression d'au moins 0,1 MPa.

37. Procédé selon l'une quelconque des revendications précédentes, dans lequel la variation de pression pendant au moins 10 % de la durée de l'étape de dépôt est inférieure à 1,0 MPa par minute.

38. Procédé selon l'une quelconque des revendications précédentes, dans lequel la demi-vie (t_{1/2P}) de la réduction de pression pendant au moins 50 % de la durée de l'étape de dépôt est sensiblement constante.

39. Procédé selon l'une quelconque des revendications précédentes, dans lequel la réduction de pression au cours des derniers 10 % de la durée de l'étape de dépôt est inférieure à 2,0 MPa par minute.

40. Procédé selon l'une quelconque des revendications précédentes, dans lequel la pression dans le réacteur est réduite de 2 bars à 1 bar aussi lentement ou plus lentement que la réduction de 3 bars à 2 bars.

41. Procédé selon l'une quelconque des revendications précédentes, dans lequel le substrat polymère solide est soumis à un prétraitement avant l'étape d'application du revêtement, le prétraitement comprenant une étape d'extraction, l'étape d'extraction comprenant l'extraction d'huiles et d'eau du substrat polymère solide.

42. Procédé selon la revendication 41, dans lequel l'étape d'extraction est exécutée en soumettant le substrat polymère solide à un ou plusieurs des traitements suivants: un traitement thermique, un traitement sous vide, un traitement avec un solvant supercritique et un traitement au dioxyde de carbone liquide.

43. Procédé selon l'une quelconque des revendications 41 et 42, dans lequel au moins 0,05 % en poids du substrat polymère solide sont extraits pendant l'étape d'extraction.

44. Procédé selon l'une quelconque des revendications précédentes, dans lequel le substrat polymère solide est soumis à un post-traitement thermique à une température qui est comprise entre Tg et Tg - 40°C du polymère de revêtement, le post-traitement thermique étant exécuté après la fin de l'étape de dépôt.

45. Procédé selon la revendication 44, dans lequel le post-traitement thermique est exécuté pendant au moins 30 minutes.

46. Procédé selon l'une quelconque des revendications précédentes, dans lequel le revêtement déposé a une épaisseur qui est comprise entre 10⁻⁵ µg/cm² et 1 mg/cm².

47. Procédé selon l'une quelconque des revendications précédentes, dans lequel le revêtement déposé présente une épaisseur sensiblement uniforme sur la surface traitée du substrat polymère solide.

48. Article susceptible d'être obtenu en utilisant le procédé selon l'une quelconque des revendications 1 à 47.

49. Article selon la revendication 48, dans lequel l'article est un dispositif médical à utiliser en contact avec le corps humain.

50. Article selon la revendication 48, dans lequel l'article est un instrument de laboratoire.
